(19) Europäisches Patentamt

European Patent Office

Office européen des brevets

(11) **EP 2 477 582 B1**

(12) **EUROPEAN PATENT SPECIFICATION**

(45) Date of publication and mention
of the grant of the patent:
**07.06.2017  Bulletin 2017/23**

(21) Application number: **10755281.2**

(22) Date of filing: **10.09.2010**

(51) Int Cl.:
***A61F 2/915*** *(2013.01)*      ***A61F 2/88*** *(2006.01)*

(86) International application number:
**PCT/US2010/048485**

(87) International publication number:
**WO 2011/034794 (24.03.2011 Gazette 2011/12)**

(54) **METHODS FOR FORMING AN ORTHOGONAL END ON A HELICAL STENT**

VERFAHREN ZUR BILDUNG EINES ORTHOGONALEN ENDES AUF EINEM HELIKALEN STENT

PROCÉDÉS DE FORMATION D'UNE EXTRÉMITÉ ORTHOGONALE SUR UNE ENDOPROTHÈSE HÉLICOÏDALE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **18.09.2009  US 243597 P**

(43) Date of publication of application:
**25.07.2012  Bulletin 2012/30**

(73) Proprietor: **Medtronic Vascular Inc.**
**Santa Rosa, CA 95403 (US)**

(72) Inventors:
• **BLISS, Richard**
**Cloverdale**
**California 95425 (US)**
• **GOSHGARIAN, Justin**
**Santa Rosa**
**California 95405 (US)**
• **LAM, Rui**
**Santa Rosa**
**California 95403 (US)**
• **SAVAGE, Padraig**
**Ballybrit**
**Galway Ireland (IE)**
• **GRISWOLD, Erik**
**Penngrove**
**California 94951 (US)**
• **PELLEGRINI, Gianfranco**
**Santa Rosa**
**California 95405 (US)**
• **BALDWIN, Matthew**
**Santa Rosa**
**California 95403 (US)**
• **ENSIGN, Lance**
**Santa Rosa**
**California 95409 (US)**

(74) Representative: **Zimmermann & Partner**
**Patentanwälte mbB**
**Postfach 330 920**
**80069 München (DE)**

(56) References cited:
**EP-A1- 0 565 251     WO-A2-2008/049045**

EP 2 477 582 B1

**Description**

**BACKGROUND OF THE INVENTION**

Field of the Invention

The present invention is generally related to a method of manufacturing a helical stent having an orthogonal end relative to a longitudinal axis of the stent, as well as to a helical stent having an end that is orthogonal to the longitudinal axis of the stent.

Background of the Invention

A stent is typically a hollow, generally cylindrical device that is deployed in a body lumen from a radially contracted configuration into a radially expanded configuration, which allows it to contact and support a vessel wall. For example, WO 2008/049045 A2 according to its title relates to a stent with flexible hinges. EP 0565 251 A1 according to its title relates to a vascular stent. A plastically deformable stent can be implanted during an angioplasty procedure by using a delivery system that includes a balloon catheter bearing a compressed or "crimped" stent, which has been loaded onto the balloon. The stent radially expands as the balloon is inflated, forcing the stent into contact with the body lumen, thereby forming a support for the vessel wall. Deployment is effected after the stent has been introduced percutaneously, transported transluminally, and positioned at a desired location by means of the balloon catheter.

Stents may be formed from wire(s), may be cut from a tube, or may be cut from a sheet of material and then rolled into a tube-like structure. While some stents may include a plurality of connected rings that are substantially parallel to each other and are oriented substantially perpendicular to a longitudinal axis of the stent, others may include a helical coil that is wrapped around the longitudinal axis at a non-perpendicular angle. Helical stents tend to have ends that are not perpendicular to the longitudinal axis due to the pitch of the helix. To square off the ends of a helical stent, the last turn at either end may include a wave form that includes waves of varying amplitudes. However, by varying the amplitudes of the waves, the stent may exhibit non-uniform behavior as the stent is crimped onto a balloon and/or expanded at the deployment site.

**SUMMARY OF THE INVENTION**

It is desirable to provide a helical stent that is configured to contract and expand more uniformly, so that a "dog bone" effect during expansion may be substantially eliminated.

It is an aspect of the present invention to provide a method of manufacturing a stent. The method includes forming a wave form having a plurality of struts and a plurality of crowns. Each crown connects two adjacent struts. The wave form has a central portion and two end portions located on opposite sides of the central portion. In an embodiment, some of the struts located in the end portions may have lengths longer than an average length of all of the struts of the wave form. In an embodiment, some of the struts located in the end portions may have lengths shorter than an average length of all of the struts of the wave form. The method includes wrapping the wave form about a longitudinal axis to define a plurality of turns so that a first turn is oriented at an angle relative to the longitudinal axis, a second turn is at a first pitch angle that is less than the angle that the first turn is disposed relative to the longitudinal axis, a third turn is at a second pitch angle that is less than the first pitch angle, and a fourth turn is at a third pitch angle that is less than the second pitch angle. In an embodiment, the first turn is substantially perpendicular to the longitudinal axis, i.e., the angle that the first turn is disposed relative to the longitudinal axis is about 90°. In an embodiment, the first turn is not substantially perpendicular to the longitudinal axis and instead has a pitch angle that is greater than 90°, and the second turn is at a pitch angle that is less than the pitch angle of the first turn, and so on.

It is an aspect of the present invention to provide a stent that includes a wave form comprising a plurality of struts and a plurality of crowns. Each crown connects two adjacent struts within the wave form. The wave form is wrapped around a longitudinal axis to define a central portion and two end portions located on opposite sides of the central portion. The central portion comprises a plurality of turns oriented at a first pitch angle relative to the longitudinal axis. The end portions each comprise a plurality of turns oriented at different pitch angles, and an end turn oriented substantially perpendicular to the longitudinal axis. The different pitch angles of the end portions are between the first pitch angle and about 90°. In an embodiment, the end turn is not oriented substantially perpendicular to the longitudinal axis and instead has a pitch angle that is greater than 90°, and the turns in the end portions have pitch angles that gradually transition from the pitch angle of the end turn to the first pitch angle.

## BRIEF DESCRIPTION OF THE DRAWINGS

Embodiments of the invention will now be described, by way of example only, with reference to the accompanying schematic drawings in which corresponding reference symbols indicate corresponding parts, and in which:

Figure 1 schematically depicts a stent according to an embodiment of the present invention in an unrolled configuration;

Figure 2 schematically depicts a wave form for the stent of Figure 1 before the wave form is wrapped around a longitudinal axis to define the stent of Figure 1;

Figure 3 schematically depicts a stent according to an embodiment of the present invention in an unrolled configuration;

Figure 4 schematically depicts a stent according to an embodiment of the present invention in an unrolled configuration;

Figure 5 schematically depicts an end turn of a stent according to an embodiment of the present invention in an unexpanded condition; and

Figure 6 schematically depicts the end turn of the stent of Figure 5 in an expanded condition.

## DETAILED DESCRIPTION OF EMBODIMENTS OF THE INVENTION

The following detailed description is merely exemplary in nature and is not intended to limit the invention or the application and use of the invention. Furthermore, there is no intention to be bound by any expressed or implied theory presented in the preceding technical field, background, brief summary or the following detailed description.

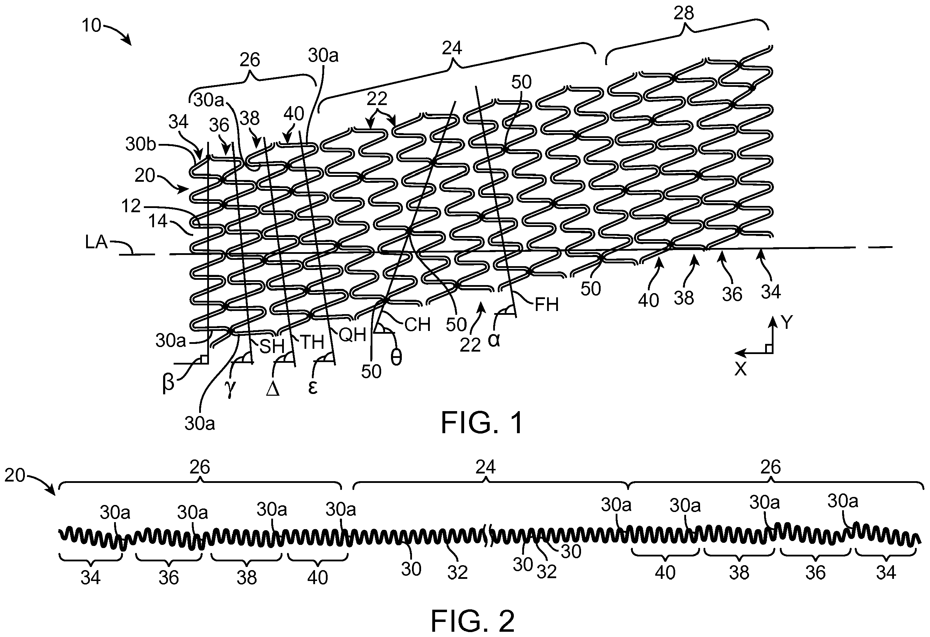

Figure 1 illustrates a stent 10 according to an embodiment of the present invention. Although the stent 10 is generally cylindrical in shape and has a longitudinal axis LA extending through the center of the stent 10, Figure 1 illustrates the stent 10 in an "unrolled" state, which may be created when the stent 10 is slit from one end to the other along an axis that is parallel to the longitudinal axis LA. The stent 10 includes a continuous wave form 20, an embodiment of which is illustrated in Figure 2, that includes a plurality of turns 22 that are created when the wave form 20 is wrapped around the longitudinal axis LA during manufacturing of the stent 10. A mandrel or rod that is aligned with the longitudinal axis LA may be used to support the wave form 20 as the wave form 20 is wrapped around the longitudinal axis LA. The stent 10 generally includes a central portion 24 and two end portions, a first end portion 26 and a second end portion 28, that are located on opposite sides of the central portion 24. In an embodiment, the first end portion 26 and the second end portion 28 may be mirror images of each other.

As illustrated in Figure 2, the wave form 20 includes a plurality of struts 30 and a plurality of crowns 32. Each crown 32 is a curved portion or turn within the wave form 20 that connects adjacent struts 30 to define the continuous wave form 20. As shown in Figure 2, the struts 30 are substantially straight portions of the wave form 20. In other embodiments, the struts 30 may be slightly bent or have other shapes, such as a sinusoidal wave, for example.

As illustrated in Figure 1, the wave form 20 is wrapped around the longitudinal axis LA at different pitches so that the wave form 20 generally defines a helical coil in the central portion 24 having a first helical angle, or first pitch angle $\alpha$, to define a first helix FH, and also defines ends that are substantially square or perpendicular with the longitudinal axis LA. As illustrated, the first end portion 26, include a first turn 34 that is wrapped about the longitudinal axis LA at an angle $\beta$ of about 90° so that the stent 10 has an end that is substantially orthogonal or perpendicular to the longitudinal axis LA. In an embodiment, the angle $\beta$ is greater than 90°.

The number of turns 22 about the longitudinal axis and the first helical angle $\alpha$ may be determined by the particular specifications of the stent 10, such as the desired unexpanded and expanded diameters and the length of the stent, as well as the size (e.g., diameter) and particular material of the wire or strip of material that may be used to create the wave form 20. The illustrated embodiments are not intended to be limiting in any way.

The first end portion 26 also includes a second turn 36 that is a continuation of the wave form 20 from the first turn 34. The second turn 36 is wrapped about the longitudinal axis LA at a second pitch angle $\gamma$ that is less than 90° but greater than the first pitch angle $\alpha$, to define a second helix SH. Additional turns may be part of the first end portion 26, such as a third turn 38, and a fourth turn 40, and may be configured to provide a more gradual transition between the first turn 34 that is wrapped about the longitudinal axis LA at about 90° and the first pitch angle $\alpha$ of the central portion 24. In the illustrated embodiment, the third turn 38 is wrapped about the longitudinal axis LA at a third pitch angle $\Delta$, which is greater than the first pitch angle $\alpha$ but less than the second pitch angle $\gamma$, to define a third helix TH, and the fourth turn 40 is wrapped about the longitudinal axis LA at a fourth pitch angle $\varepsilon$, which is greater than the first pitch angle $\alpha$ but less than the third pitch angle $\gamma$, to define a fourth helix QH. Although three transitional turns 36, 38, 40 are illustrated in the embodiment of Figure 1, more or less transitional turns may be used. The illustrated embodiment is not intended to be limiting in any way.

As illustrated, each of the turns 34, 36, 38, 40 of the first end portion 26 include struts 30 having different lengths, and some of the struts 30 have a length that is longer, labeled 30a in Figure 1, than the average length of all of the struts 30 of the stent 10. It is desirable to have the length of the longest strut 30a of any given turn 34, 36, 38, 40 to be as short as possible, yet provide the desired transition in pitch angle. The presence of the longer struts 30a in the first end portion 26 allow for the transition from the orthogonal end to the helical central portion 24, but may cause the stent 10 to expand unevenly, as compared to central portion 24, when an internal pressure is applied to the stent 10. It may be desirable to connect the crown 32 that connects a longer strut 30a within a turn 22 to a crown 32 of the next turn in order to impede the expansion of the part of the wave form 20 that contains the longer strut 30a. In an embodiment, some of the struts located in the first end portion 26 may have lengths that are shorter, labeled 30b in Figure 1, than an average length of all of the struts 30 of the stent 10.

The stent 10 also includes a plurality of connections 50 that are configured to connect selected crowns 32 of adjacent turns 22 so that when the stent is in an unexpanded condition, the plurality of connections 50 generally lie along a connection helix CH defined by a connection helical angle $\theta$ relative to the longitudinal axis LA. As illustrated in Figure 1, the connection helix CH is oriented substantially opposite to the first helix FH described above such that the connection helical angle $\theta$ is between 0° and 90° when using a coordinate system that is opposite the coordinate system depicted in Figure 1 (i.e., the positive x axis runs from left to right rather than from right to left).

The connections 50 may be created by fusing the selected crowns 32 together. As used herein, "fusing" is defined as heating the target portions of materials to be fused together, without adding any additional material, to a level where the material in the target portions flow together, intermix with one another, and form a fusion when the materials cool down to, for example, room temperature. A suitable laser may be used to create the fusion.

In an embodiment, the connections 50 may be created by welding or soldering the selected crowns 32 together. As used herein, "welding" and "soldering" are defined as heating an additional material that is separate from the selected crowns and applying the heated additional material to the selected crowns 32 so that when the additional material cools, the selected crowns 32 are welded or soldered together.

In an embodiment, the connections 50 may be created by fusing, welding, or soldering an additional piece of material (not shown) that extends between selected crowns 32. The additional piece of material may resemble a strut or a portion of a strut, and may be sized to provide spacing between the selected crowns of two adjacent turns, if desired. The illustrated embodiments are not intended to be limiting in any way.

The size of the connections 50 may also be varied according to the desired flexibility and rate of expansion for a given area of the stent 10. In general, the larger the connection 50, i.e. the larger the fusion or weld, the greater the stiffness, and the slower the rate of expansion of the stent in the area of the larger connections.

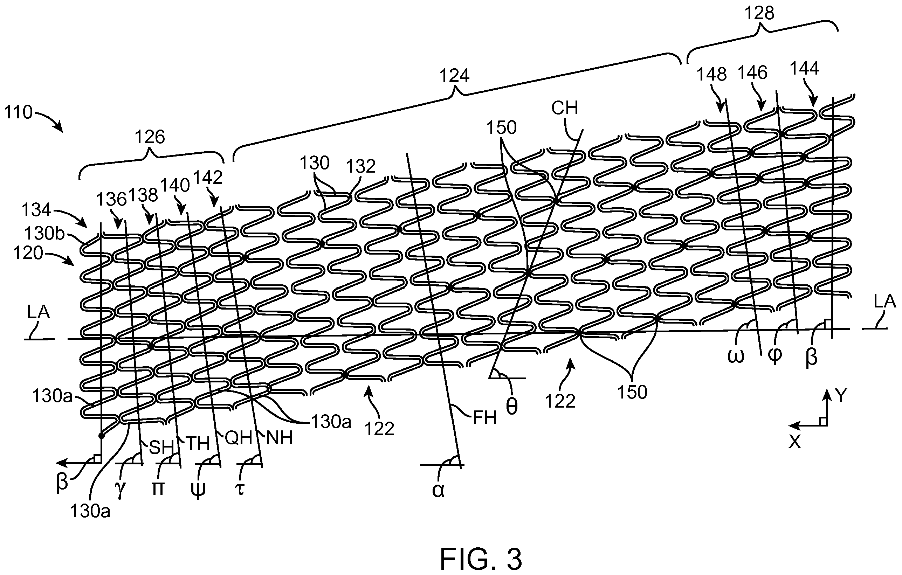

Figure 3 illustrates a stent 110 according to an embodiment of the present invention an "unrolled" state. The stent 110 includes a continuous wave form 120 that includes a plurality of turns 122 that are created when the wave form 120 is wrapped around the longitudinal axis LA during manufacturing of the stent 110. The stent 110 generally includes a central portion 124 and two end portions, a first end portion 126 and a second end portion 128, that are located on opposite sides of the central portion 124.

As illustrated in Figure 3, the wave form 120 includes a plurality of struts 130 and a plurality of crowns 132. Each crown 132 is a curved portion or turn within the wave form 120 that connects adjacent struts 130 to define the continuous wave form 120. As shown in Figure 3, the struts 130 are substantially straight portions of the wave form 120. In other embodiments, the struts 130 may be slightly bent or have other shapes, such as a sinusoidal wave, for example.

As illustrated in Figure 3, the wave form 120 is wrapped around the longitudinal axis LA at different pitches so that the wave form 120 generally defines a helical coil in the central portion 124 having a first helical angle, or first pitch angle $\alpha$, to define a first helix FH, and also defines ends that are substantially square or perpendicular with the longitudinal axis LA. As illustrated, the first end portion 126, include a first turn 134 that is wrapped about the longitudinal axis LA at an angle $\beta$ of about 90° so that the stent 110 has an end that is substantially square or perpendicular to the longitudinal axis LA. In an embodiment, the angle $\beta$ may be greater than 90°.

The number of turns 122 about the longitudinal axis and the first helical angle $\alpha$ may be determined by the particular specifications of the stent 110, such as the desired unexpanded and expanded diameters and the length of the stent, as well as the size (e.g., diameter) and particular material of the wire or strip of material. The illustrated embodiments are not intended to be limiting in any way.

The first end portion 126 also includes a second turn 136 that is a continuation of the wave form 120 from the first turn 134. The second turn 136 is wrapped about the longitudinal axis LA at a second pitch angle $\rho$ that is less than 90° but greater than the first pitch angle $\alpha$, to define a second helix SH. Additional turns may be part of the first end portion 126, such as a third turn 138, and a fourth turn 140, and a fifth turn 142, and may be configured to provide a more gradual transition between the first turn 134 that is wrapped about the longitudinal axis LA at about 90° and the first pitch angle $\alpha$ of the central portion 124. In the illustrated embodiment, the third turn 138 is wrapped about the longitudinal axis LA at a third pitch angle $\pi$, which is greater than the first pitch angle $\alpha$ but less than the second pitch angle $\rho$, to define a third helix TH. The fourth turn 140 is wrapped about the longitudinal axis LA at a fourth pitch angle $\psi$, which is greater than the first pitch angle $\alpha$ but less than the third pitch angle $\pi$, to define a fourth helix QH. The fifth turn 142 is wrapped about the longitudinal axis LA at a fifth pitch angle $\tau$, which is greater than the first pitch angle $\alpha$ but less than the fourth pitch angle $\pi$, to define a fifth helix NH.

As illustrated, each of the turns 134, 136, 138, 140, 142 of the first end portion 126 include struts 130 having different lengths, and some of the struts 130 have a length that is longer, labeled 130a in Figure 3, than the average length of all of the struts 130 of the stent 110. It is desirable to have the length of the longest strut 130a of any given turn 134, 136, 138, 140, 142 to be as short as possible, yet provide the desired transition in pitch angle.

The presence of the longer struts 130a in the first end portion 126 allow for the transition from the orthogonal end to the helical central portion 124, but may cause the stent 110 to expand unevenly, as compared to central portion 124, when an internal pressure is applied to the stent 110. It may be desirable to connect the crown 132 that connects a longer strut 130a within a turn 122 to a crown 132 of the next turn in order to stiffen that area and impede the expansion of the part of the wave form 120 that contains the longer strut 130a. In an embodiment, some of the struts located in the first end portion 126 may have lengths that are shorter, labeled 130b in Figure 3, than an average length of all of the struts 130 of the stent 110. By using additional transition turns in the end portion 126 of the stent 110 illustrated in Figure 3, as compared to the end portion 26 of the stent 10 illustrated in Figure 1, the longer struts 130a illustrated in Figure 3 may be shorter than the longer struts 30a illustrated in Figure 1.

The stent 110 also includes a plurality of connections 150 that are configured to connect selected crowns 312 of adjacent turns 122 so that when the stent is in an unexpanded condition, the plurality of connections 150 generally lie along a connection helix CH defined by a connection helical angle $\theta$ relative to the longitudinal axis LA. As illustrated in Figure 3, the connection helix CH is oriented substantially opposite to the first helix FH described above such that the connection helical angle $\theta$ is between 0° and 90° when using a coordinate system that is opposite the coordinate system depicted in Figure 3 (i.e., the positive x axis runs from left to right rather than from right to left).

As also illustrated in Figure 3, the end portions 126, 128 are not necessarily mirror images of each other. The end portion 126 includes a total of five turns 134, 136, 138, 140, 142, while the end portion 128 only includes three turns 144, 146, 148. The end turn 144 of the end portion 128 has a pitch angle $\beta$ of about 90° so that the end of the end portion 128 is substantially perpendicular to the longitudinal axis LA. In an embodiment, the end turn of the end portion 128 has a pitch angle that is greater than 90°. The next turn 146 is wrapped about the longitudinal axis LA at a pitch angle $\varphi$, which is less than the angle $\beta$ (i.e., ~90°), but greater than the first pitch angle $\alpha$ of the central portion 124. The next turn 148 is wrapped about the longitudinal axis LA at a pitch angle w, which is less than the pitch angle $\varphi$ of the turn 146, but greater than the first pitch angle $\alpha$ of the central portion 124.

Of course, any number of transition turns may be in each end portion 126, 128 to transition the helix of the central portion 124 to an orthogonal end. In an embodiment, the central portion of stent consists of a series of transitions so that the entire stent is made up of transitions and each turn includes struts of different lengths, and no two adjacent turns have the same pitch angle. The illustrated embodiments are not intended to be limiting in any way.

The change in pitch angle from turn to turn within a transition, such as within the end portions 26, 28, 126, 128 discussed above and illustrated in Figures 1 and 3, is constant and can be calculated as the difference between the pitch angle of the end turn and the pitch angle of the central portion divided by the number of transition turns in between the end turn and the central portion, or:

$$\text{change in pitch angle} = (\beta - \alpha)/\text{\# transition turns} \qquad (1)$$

Using equation 1, the various pitch angles for the turns 36, 38, 40 of Figure 1 and for the turns 136, 138, 140, 142, 146, 148 of Figure 3 may be calculated. In an embodiment, the change in pitch angle between adjacent turns may not be constant.

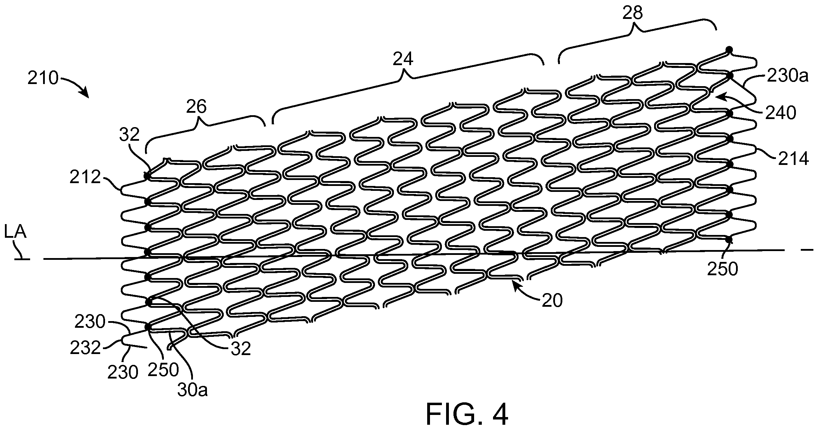

Figure 4 illustrates and embodiment of a stent 210 that is substantially similar to the stent 10 of Figure 1, but with additional end segments 212, 214 that are connected to each end portion 26, 28 of the wave form 20. The end segments 212, 214 may be formed separate from the wave form 20 and may be connected to the end portions 26, 28 of the wave form 20 after the wave form 20 has been wrapped around the longitudinal axis LA. The end segments 212, 214 may be formed from a wire or other suitable strip of material, or may be cut from a tube or sheet of material and rolled into a ring-like structure.

As illustrated in Figure 4, the end segment 212 includes a plurality of struts 230 having substantially the same length and a plurality of crowns 232 having substantially same size. Each crown 232 connects adjacent struts 230 so as to form a continuous ring having a constant length along the longitudinal axis LA. The end segment 212 may be connected to the wave form 20 portion of the stent 210 with a plurality of connections 250. In the illustrated embodiment, every crown 32 of the end portion 26 of the wave form 20 is connected to a corresponding crown 232 of the end segment 212. The end segment 212 may assist in controlling expansion of the areas of the end portion 26 that include the longer struts 30a so that the end of the stent 210 expands more uniformly with the rest of the stent 210.

The end segment 214 may have substantially the same design as the end segment 212, or the end segment 214 may have a different design to compensate for the end of the wave form 20, as illustrated in Figure 4. For example, the end segment 214 may include a longer strut 230a to bridge a gap 240 that is created due to the end of the wave form 20. Other configurations of the end segment 212, 214 in accordance with embodiments of the present invention are contemplated, and the illustrated embodiment is not intended to be limiting in any way.

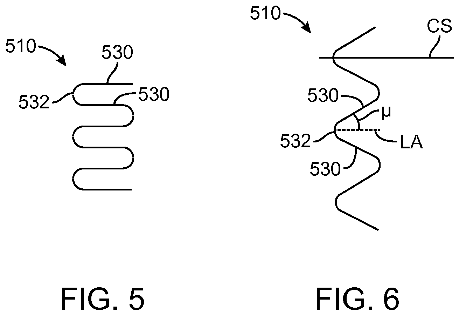

In addition to having the ends of the stent orthogonal to the longitudinal axis LA of the stent, as well as having substantially uniform expansion properties upon deployment, it is also desirable for the ends of the stent to be well opposed to the vessel wall when the stent is deployed at the target deployment site. It has been found that the greater the angle of strut deployment, the greater the propensity of the deployed struts to protrude outwardly. Figure 5 illustrates an end turn 510 of a stent in accordance with an embodiment of the invention. The end turn 510 includes a plurality of struts 530 and a plurality of crowns 532 that connect adjacent struts 530 of the end turn 510 to each other. In an embodiment, the end segment 212 of the stent 210 of Figure 4 includes the end turn 510 of Figure 5.

The struts 530 and the crowns 532 are configured to provide a deployment angle $\mu$, illustrated in Figure 6, of at least 40°. The deployment angle $\mu$ is defined as the angle between the longitudinal axis LA of the stent and one of the struts 530 when the crowns 132 and struts 130 have started to plastically deform so that when the internal pressure is relieved from the stent, the crowns 532 and struts 530 remain in the same position and do not contract towards the longitudinal axis LA. It has been found that designing the crowns 532 and struts 530 to have a deployment angle of at least 40° allows the struts 530 to protrude outwardly from the outer circumferential surface of the rest of the stent (schematically depicted in Figure 6 as line CS), which may allow the end of the stent to be well opposed to the vessel wall when the stent is deployed at the target deployment site.

The embodiments of the stents discussed above may be formed from a wire or a strip of suitable material. In certain embodiments, the stents may be formed, i.e., etched or cut, from a thin tube of suitable material, or from a thin plate of suitable material and rolled into a tube. Suitable materials for the stent include but are not limited to stainless steel, iridium, platinum, gold, tungsten, tantalum, palladium, silver, niobium, zirconium, aluminum, copper, indium, ruthenium, molybdenum, niobium, tin, cobalt, nickel, zinc, iron, gallium, manganese, chromium, titanium, aluminum, vanadium, and carbon, as well as combinations, alloys, and/or laminations thereof. For example, the stent may be formed from a cobalt alloy, such as L605 or MP35N®, Nitinol (nickel-titanium shape memory alloy), ABI (palladium-silver alloy), Elgiloy® (cobalt-chromium-nickel alloy), etc. It is also contemplated that the stent may be formed from two or more materials that are laminated together, such as tantalum that is laminated with MP35N®. The stents may also be formed from wires having concentric layers of different materials. Embodiments of the stent may also be formed from hollow tubes, or tubes that have been filled with other materials. The aforementioned materials and laminations are intended to be examples and are not intended to be limiting in any way.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient roadmap for implementing an exemplary embodiment of the invention, it being understood that various changes may be made in the function and arrangement of members described in an exemplary embodiment without departing from the scope of the invention as set forth in the appended claims.

## Claims

1. A method of manufacturing a stent (10), the method comprising:

   forming a wave form (20) having a plurality of struts (30) and a plurality of crowns (32), each crown connecting two adjacent struts, the wave form having a central portion (24) and two end portions (26, 28) located on opposite sides the central portion, some of the struts located in the end portions have lengths longer and/or shorter than an average length of all of the struts of the wave form; and
   wrapping the wave form about a longitudinal axis (LA) to define a plurality of turns (34, 36, 38, 40, 22) so that an end turn is disposed at an angle relative to the longitudinal axis, a second turn (36) is at a first pitch angle that is less than the angle that the end turn is disposed relative to the longitudinal axis, a third turn (38) is at a second pitch angle that is less than the first pitch angle, and a fourth turn (40) is at a third pitch angle that is less than the second pitch angle.

2. The method of claim 1, wherein the angle that the end turn is disposed relative to the longitudinal axis is about 90°; or wherein the angle that the end turn is disposed relative to the longitudinal axis is greater than 90°.

3. The method of claim 1, wherein the difference between the first pitch angle and the second pitch angle is substantially the same as the difference between the second pitch angle and the third pitch angle.

4. The method of claim 1, further comprising wrapping the wave form about the longitudinal axis so that a plurality of turns are at the third pitch angle and form a central portion of the stent, the central portion of the wave form corresponding to the central portion of the stent; or further comprising connecting selected crowns of adjacent turns with connections.

5. The method of claim 4, wherein the connecting comprises fusing the selected crowns of adjacent turns to each other; or
wherein the connecting comprises welding the selected crowns of adjacent turns to each other; or
wherein the connecting comprises aligning at least some of the connections along a connection axis that defines a helix oriented in an opposite direction as the third pitch angle.

6. The method of claim 1, wherein the end portions comprise struts having different lengths to accommodate for the different pitch angles, wherein typically the method is further comprising connecting an end segment (212, 214) to selected crowns of the first turn of the wave form.

7. A stent (10) comprising:

a wave form comprising a plurality of struts (39) and a plurality of crowns (32), each crown connecting two adjacent struts within the wave form, the wave form being wrapped around a longitudinal axis (LA) to define a central portion (24) and two end portions(26, 28) located on opposite sides of the central portion, the central portion comprising a plurality of turns (22) oriented at a first pitch angle relative to the longitudinal axis, **characterised in that** the end portions each comprising a plurality of turns (34, 36, 38, 40) oriented at different pitch angles, and an end turn oriented at an angle relative to the longitudinal axis, the different pitch angles of the end portions being between the first pitch angle and the angle of the end turn relative to the longitudinal axis.

8. The stent according to claim 7, wherein the angle that the end turn is oriented is about 90°; or wherein the angle that the end turn is oriented is greater than 90°.

9. The stent according to claim 7, wherein each end portion comprises at least two turns in addition to the end turn, wherein a first turn of the end portion is connected to the central portion and is oriented at a second pitch angle, and wherein a second turn of the end portion is connected to the first turn and is oriented at a third pitch angle that is greater than the second pitch angle.

10. The stent according to claim 9, wherein the difference between the second pitch angle and the first pitch angle is substantially equal to the difference between the third pitch angle and the second pitch angle.

11. The stent according to claim 7, further comprising a plurality of connections (50) that connect selected crowns from adjacent turns of the wave form.

12. The stent according to claim 11, wherein the connections are fusions; or wherein the connections are welds.

13. The stent according to claim 11, wherein at least some of the connections are aligned along a helix at a pitch angle that is opposite the first pitch angle.

14. The stent according to claim 7, wherein each end portion comprises at least one strut that is longer and/or shorter than an average length of the plurality of struts to accommodate for the different pitch angles.

15. The stent according to claim 14, further comprising an end segment (212, 214) connected to the end turn, the end segment oriented substantially perpendicular to the longitudinal axis and comprising a plurality of crowns and a plurality of struts, the plurality of struts having substantially the same length;
wherein typically the end segment is configured to have a deployment angle of greater than about 40° relative to the longitudinal axis.

**Patentansprüche**

1. Verfahren zur Herstellung eines Stents (10), wobei das Verfahren umfasst:

    Ausbilden einer Wellenform (20) mit einer Vielzahl von Stegen (30) und einer Vielzahl von Kronen (32), wobei jede Krone zwei angrenzende Streben verbindet, wobei die Wellenform einen Mittelabschnitt (24) und zwei auf entgegengesetzten Seiten des Mittelabschnitts angeordnete Endabschnitte (26, 28) aufweist, wobei manche der in den Endabschnitten angeordneten Streben Längen aufweisen, die länger und/oder kürzer als eine durchschnittliche Länge aller Streben der Wellenform sind; und
    Wickeln der Wellenform um eine Längsachse (LA), um eine Vielzahl von Kurven (34, 36, 38, 40, 22) zu definieren, sodass eine Endkurve in einem Winkel relativ zu der Längsachse angeordnet ist, sich eine zweite Kurve (36) in einem ersten Neigungswinkel befindet, der kleiner als der Winkel ist, in dem die Endkurve relativ zu der Längsachse angeordnet ist, sich eine dritte Kurve (38) in einem zweiten Neigungswinkel befindet, der kleiner als der erste Neigungswinkel ist, und sich eine vierte Kurve (40) in einem dritten Neigungswinkel befindet, der kleiner als der zweite Neigungswinkel ist.

2. Verfahren nach Anspruch 1, wobei der Winkel, in dem die Endkurve relativ zu der Längsachse angeordnet ist, etwa 90° beträgt; oder
    wobei der Winkel, in dem die Endkurve relativ zu der Längsachse angeordnet ist, größer als 90° ist.

3. Verfahren nach Anspruch 1, wobei die Differenz zwischen dem ersten Neigungswinkel und dem zweiten Neigungswinkel im Wesentlichen der Differenz zwischen dem zweiten Neigungswinkel und dem dritten Neigungswinkel entspricht.

4. Verfahren nach Anspruch 1, ferner umfassend ein Wickeln der Wellenform um die Längsachse, sodass sich eine Vielzahl von Kurven in dem dritten Neigungswinkel befindet und einen Mittelabschnitt des Stents bildet, wobei der Mittelabschnitt der Wellenform dem Mittelabschnitt des Stents entspricht; oder ferner umfassend ein Verbinden ausgewählter Kronen angrenzender Kurven mit Verbindungen.

5. Verfahren nach Anspruch 4, wobei das Verbinden ferner ein Fusionieren der ausgewählten Kronen angrenzender Kurven aneinander umfasst; oder
    wobei das Verbinden ein Verschweißen der ausgewählten Kronen angrenzender Kurven aneinander umfasst; oder
    wobei das Verbinden ein Ausrichten zumindest mancher der Verbindungen entlang einer Verbindungsachse umfasst, welche eine Helix definiert, die in eine entgegengesetzte Richtung als der dritte Neigungswinkel ausgerichtet ist.

6. Verfahren nach Anspruch 1, wobei die Endabschnitte Streben mit verschiedenen Längen umfassen, um die verschiedenen Neigungswinkel zu ermöglichen, wobei das Verfahren typischerweise ein Verbinden eines Endsegmentes (212, 214) mit ausgewählten Kronen der ersten Kurve der Wellenform umfasst.

7. Stent (10), umfassend:

    eine Wellenform, umfassend eine Vielzahl von Streben (39) und eine Vielzahl von Kronen (32), wobei jede Krone zwei angrenzende Streben in der Wellenform verbindet, wobei die Wellenform um eine Längsachse (LA) gewickelt ist, um einen Mittelabschnitt (24) und zwei Endabschnitte (26, 28) zu definieren, die sich auf entgegengesetzten Seiten des Mittelabschnitts befinden,
    wobei der Mittelabschnitt eine Vielzahl von Kurven (22) umfasst, die in einem ersten Neigungswinkel relativ zu der Längsachse ausgerichtet ist,
    **dadurch gekennzeichnet, dass** die Endabschnitte jeweils eine Vielzahl von in verschiedenen Neigungswinkeln ausgerichteten Kurven (34, 36, 38, 40) und eine Endkurve, die in einem Winkel relativ zu der Längsachse ausgerichtet ist, umfassen,
    wobei die verschiedenen Neigungswinkel der Endabschnitte zwischen dem ersten Neigungswinkel und dem Winkel der Endkurve relativ zu der Längsachse sind.

8. Stent nach Anspruch 7, wobei der Winkel, in dem die Endkurve ausgerichtet ist, etwa 90° beträgt; oder wobei der Winkel, in dem die Endkurve ausgerichtet ist, größer als 90° ist.

9. Stent nach Anspruch 7, wobei jeder Endabschnitt zusätzlich zu der Endkurve mindestens zwei Kurven umfasst, wobei eine erste Kurve des Endabschnitts mit dem Mittelabschnitt verbunden und in einem zweiten Neigungswinkel ausgerichtet ist, und wobei eine zweite Kurve des Endabschnitts mit der ersten Kurve verbunden und in einem dritten Neigungswinkel, der größer als der zweite Neigungswinkel ist, ausgerichtet ist.

10. Stent nach Anspruch 9, wobei die Differenz zwischen dem zweiten Neigungswinkel und dem ersten Neigungswinkel im Wesentlichen der Differenz zwischen dem dritten Neigungswinkel und dem zweiten Neigungswinkel entspricht.

11. Stent nach Anspruch 7, ferner umfassend eine Vielzahl von Verbindungen (50), die ausgewählte Kronen angrenzender Kurven der Wellenform verbinden.

12. Stent nach Anspruch 11, wobei die Verbindungen Fusionen sind; oder wobei die Verbindungen Schweißnähte sind.

13. Stent nach Anspruch 11, wobei zumindest manche der Verbindungen entlang einer Helix in einem Neigungswinkel ausgerichtet sind, der dem ersten Neigungswinkel entgegengesetzt ist.

14. Stent nach Anspruch 7, wobei jeder Endabschnitt mindestens eine Strebe umfasst, die länger und/oder kürzer als eine durchschnittliche Länge der Vielzahl von Streben ist, um die verschiedenen Neigungswinkel zu ermöglichen.

15. Stent nach Anspruch 14, ferner umfassend ein mit der Endkurve verbundenes Endsegment (212, 214), wobei das Endsegment im Wesentlichen senkrecht zu der Längsachse ausgerichtet ist und eine Vielzahl von Kronen und eine Vielzahl von Streben umfasst, wobei die Vielzahl von Streben im Wesentlichen die gleiche Länge aufweist; wobei das Endsegment typischerweise derart ausgelegt ist, dass es einen Zuführungswinkel von über etwa 40° relativ zu der Längsachse aufweist.


## Revendications

1. Procédé de fabrication d'une endoprothèse (10), le procédé comprenant :

   la formation d'une forme ondulée (20) ayant une pluralité de branches (30) et une pluralité de sommets (32), chaque sommet reliant deux branches adjacentes, la forme ondulée ayant une partie centrale (24) et deux parties terminales (26, 28) situées sur des côtés opposés de la partie centrale, dont certaines des branches situées dans les parties terminales ont des longueurs plus longues et/ou plus courtes qu'une longueur moyenne de toutes les branches de la forme ondulée ; et
   l'enroulement de la forme ondulée autour d'un axe longitudinal (LA) pour définir une pluralité de tours (34, 36, 38, 40, 22) de sorte qu'un tour terminal est disposé à un angle par rapport à l'axe longitudinal, un deuxième tour (36) est à un premier angle de pas qui est inférieur à l'angle selon lequel le tour terminal est disposé par rapport à l'axe longitudinal, un troisième tour (38) est à un deuxième angle de pas qui est inférieur au premier angle de pas, et un quatrième tour (40) est à un troisième angle de pas qui est inférieur au deuxième angle de pas.

2. Procédé selon la revendication 1, dans lequel l'angle selon lequel le tour terminal est disposé par rapport à l'axe longitudinal est d'environ 90° ; ou
   dans lequel l'angle selon lequel le tour terminal est disposé par rapport à l'axe longitudinal est supérieur à 90°.

3. Procédé selon la revendication 1, dans lequel la différence entre le premier angle de pas et le deuxième angle de pas est sensiblement la même que la différence entre le deuxième angle de pas et le troisième angle de pas.

4. Procédé selon la revendication 1, comprenant en outre l'enroulement de la forme ondulée autour de l'axe longitudinal de sorte qu'une pluralité de tours est au troisième angle de pas et forme une partie centrale de l'endoprothèse, la partie centrale de la forme ondulée correspondant à la partie centrale de l'endoprothèse ; ou comprenant en outre le raccord de sommets sélectionnés de tours adjacents avec des raccordements.

5. Procédé selon la revendication 4, dans lequel le raccord comprend le fusionnement de sommets sélectionnés de tours adjacents les uns avec les autres ; ou
   dans lequel le raccord comprend le soudage des sommets sélectionnés de tours adjacents les uns avec les autres ; ou dans lequel le raccord comprend l'alignement d'au moins certains des raccordements le long d'un axe de raccordement qui définit une hélice orientée dans une direction opposée comme troisième angle de pas.

6. Procédé selon la revendication 1, dans lequel les parties terminales comprennent des branches ayant différentes longueurs pour recevoir les différents angles de pas, dans lequel habituellement, le procédé comprend en outre le raccord d'un segment terminal (212, 214) à des sommets sélectionnés du premier tour de la forme ondulée.

7. Endoprothèse (10) comprenant :

une forme ondulée comprenant une pluralité de branches (39) et une pluralité de sommets (32), chaque sommet raccordant deux branches adjacentes dans la forme ondulée, la forme ondulée étant enroulée autour d'un axe longitudinal (LA) de façon à définir une partie centrale (24) et deux parties terminales (26, 28) situées sur des côtés opposés de la partie centrale, la partie centrale comprenant une pluralité de tours (22) orientée à un premier angle de pas par rapport à l'axe longitudinal,
**caractérisée en ce que** les parties terminales comprenant chacune une pluralité de tours (34, 36, 38, 40) orientée à différents angles de pas et un tour terminal orienté à un angle par rapport à l'axe longitudinal ;
les différents angles de pas des parties terminales étant entre le premier angle de pas et l'angle de tour terminal par rapport à l'axe longitudinal.

8. Endoprothèse selon la revendication 7, dans laquelle l'angle selon lequel le tour terminal est orienté est d'environ 90°; ou dans laquelle l'angle selon lequel le tour terminal est orienté est supérieur à 90°.

9. Endoprothèse selon la revendication 7, dans laquelle chaque partie terminale comprend au moins deux tours en plus du tour terminal, dans laquelle un premier tour de la partie terminale est raccordé à la partie centrale et est orienté à un deuxième angle de pas, et dans laquelle un deuxième tour de la partie terminale est raccordé au premier tour et est orienté à un troisième angle de pas qui est supérieur au deuxième angle de pas.

10. Endoprothèse selon la revendication 9, dans laquelle la différence entre le deuxième angle de pas et le premier angle de pas est sensiblement égale à la différence entre le troisième angle de pas et le deuxième angle de pas.

11. Endoprothèse selon la revendication 7, comprenant en outre une pluralité de raccordements (50) qui raccorde des sommets sélectionnés de tours adjacents de la forme ondulée.

12. Endoprothèse selon la revendication 11, dans laquelle les raccordements sont des fusions ; ou dans laquelle les raccordements sont des soudures.

13. Endoprothèse selon la revendication 11, dans laquelle au moins certains des raccordements sont alignés le long d'une hélice à un angle de pas qui est opposé au premier angle de pas.

14. Endoprothèse selon la revendication 7, dans laquelle chaque partie terminale comprend au moins une branche qui est plus longue et/ou plus courte qu'une longueur moyenne de la pluralité de branches pour recevoir les différents angles de pas.

15. Endoprothèse selon la revendication 14, comprenant en outre un segment terminal (212, 214) raccordé au tour terminal, le segment terminal étant orienté de façon sensiblement perpendiculaire à l'axe longitudinal et comprenant une pluralité de sommets et une pluralité de branches, la pluralité de branches ayant sensiblement la même longueur ; dans laquelle habituellement le segment terminal est configuré de façon à avoir un angle de déploiement supérieur à environ 40° par rapport à l'axe longitudinal.

FIG. 1

FIG. 2

FIG. 3

FIG. 4

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008049045 A2

- EP 0565251 A1